# FASCICULE DE BREVET EUROPEEN

(11) **EP 0 778 048 B1**
(45) Date de publication et mention de la délivrance du brevet: **10.09.2003**
(21) Numéro de dépôt: 96402644.7
(22) Date de dépôt: 06.12.1996
(51) Int. Cl.: A61N 1/05

(54) **Nécessaire pour la passivation in situ d'une sonde cardiaque**
Gerät zur in situ Passivierung einer Herzsonde
Device for passivating in situ the tip of an cardiac probe

(30) Priorité: 08.12.1995 FR 9514580
(43) Date de publication de la demande: 11.06.1997
(73) Titulaire: ELA MEDICAL (Société anonyme), 92541 Montrouge (FR)
(72) Inventeur: Pioger, Guy, 75015 Paris (FR); Ollivier, Jean-François, 78280 Guyancourt (FR); Ripart, Alain, 91190 Gif sur Yvette (FR)
(74) Mandataire: Dupuis-Latour, Dominique

(56) Documents cités:
- EP-A- 0 442 444
- EP-A- 0 589 182
- EP-A- 0 622 089
- EP-A- 0 635 281
- WO-A-95/11056
- US-A- 4 917 104
- US-A- 5 487 757

## Description

L'invention concerne la passivation *in situ* d'un embout de sonde de dispositif médical implantable actif, notamment d'une sonde de stimulateur cardiaque.

Elle s'applique plus spécifiquement à un type de sonde comportant une gaine isolante creuse en matériau souple avec un conducteur interne terminé à son extrémité distale par une électrode, la sonde comportant sur toute sa longueur un canal axial contenant, à son extrémité distale dans la région voisine de l'électrode, un élément rapporté, notamment un fil ou lame métallique de conformation ou de renforcement de l'extrémité de sonde, cet élément étant de rigidité supérieure à celle de l'ensemble gaine et conducteur. On connaît également d'autres configurations où l'élément rapporté est situé dans la gaine, mais à l'extérieur du canal axial.

Par commodité, on appellera par la suite "lame" ou "lame métallique" l'élément de renforcement relativement rigide, bien que ce terme ne soit pas limitatif, l'élément pouvant avoir par exemple une forme de fil ou être réalisé en un matériau autre que métallique

En effet, on a récemment constaté que certaines sondes de ce type présentaient, sur le long terme, un risque de fracture de la lame. Or la rupture de cette lame, compte tenu de sa raideur relative et de la fragilité du matériau de la gaine (généralement un polyuréthanne) peut provoquer un percement de la gaine et une protrusion de la lame, avec risque de perforation du coeur.

En outre, même si la lame est intacte, du fait de sa forme recourbée, une tentative d'explantation de la sonde par traction risque d'entraîner une déchirure de la paroi cardiaque ou artérielle.

Ces risques ont été décrits et étudiés dans diverses publications, notamment par J. C. Daubert et coll., *Que faire face aux défaillances des sondes* Telectronics Accufix ?, Stimucoeur 1995, tome 23, n° 1 pp. 27-28, B. Dodinot, *Ouvrons l'OEil*, Stimucoeur 1995, tome 23, n° 1 pp. 39-44, M. A. Lloyd et coll., *Atrial* "J" *Pacing Lead Retention Wire Fracture: Radiographic Assessment, Incidence of Fracture, and Clinical Management,* PACE, Vol. 18, Mai 1995, pp. 958-964, et V. Parsonnet, *The Rétention Wire Fix,* PACE, Vol. 18, Mai 1995, pp. 955-997.

L'un des buts de la présente invention est de "passiver" la sonde, c'est-à-dire à immobiliser l'élément potentiellement dangereux en enrobant cet élément de manière à protéger la gaine de la sonde en cas de fracture de la lame, empêchant de cette façon toute protrusion de celui-ci au travers de la gaine.

La sonde ainsi rendue inoffensive pourra être soit laissée sur place, soit explantée sans risque de conséquences dommageables si la lame métallique venait à se briser en cours d'intervention.

L'invention a ainsi pour objet un nécessaire de passivation d'une sonde cardiaque installée chez un patient, cette sonde comprenant une extrémité proximale et une extrémité distale, un conducteur interne, une gaine externe, un volume interne s'étendant de l'extrémité proximale à l'extrémité distale, un élément rapporté disposé à l'extrémité distale et dont la rigidité est supérieure à celle, combinée, de la gaine et du conducteur.

Le nécessaire comporte, comme cela est connu du EP-A-0 589 182, un matériau durcissable susceptible de passer d'un état liquide à un état solide; une seringue avec une chambre d'injection pour recevoir le matériau durcissable dans son état fluide, et un orifice de sortie ; un tube d'injection avec une extrémité proximale et une extrémité distale, ce tube d'injection pouvant être inséré dans le volume intérieur de la sonde. De façon caractéristique de l'invention, le nécessaire comprend un raccord en T avec une première branche, une deuxième branche et une troisième branche, la deuxième et la troisième branche possédant chacune une valve permettant d'ouvrir et de fermer cette branche ; où la première branche du raccord en T se raccorde à frottement avec l'extrémité proximale du tube d'injection, la deuxième branche du raccord en T est conçue pour être raccordée à une source de vide et la troisième branche du raccord en T se raccorde à l'orifice de sortie de la seringue, et où la première et la seconde valves peuvent être actionnées pour créer un vide dans le volume intérieur de la sonde et aspirer ainsi le matériau durcissable jusqu'à l'extrémité distale de la sonde.

Selon un certain nombre de caractéristiques avantageuses :
― le nécessaire comprend en outre un mandrin dimensionné de manière à lui permettre de passer dans le volume intérieur de la gaine ;
― le nécessaire comprend en outre une ligature permettant de raccorder la gaine au tube d'injection ;
― le tube d'injection comporte en outre une marque correspondant à une distance entre cette marque et l'extrémité distale du tube ;
― le nécessaire comprend en outre une longueur de tube, de préférence transparente, pour relier la deuxième branche du raccord en T à une source de vide ;
― le nécessaire comprend en outre un mandrin dimensionné de manière à lui permettre de passer dans le volume intérieur du tube d'injection.

D'autres caractéristiques et avantages de l'invention apparaîtront à la lecture de la description détaillée ci-dessous, faite en référence aux dessins annexés.

La figure 1 est une vue en coupe schématique de la sonde et de l'appareillage permettant d'opérer sa passivation.

La figure 2 est une vue perspective illustrant l'une des étapes du procédé.

La figure 3 est une coupe agrandie du détail repéré III sur la figure 1, après injection et réticulation du matériau durcissable.

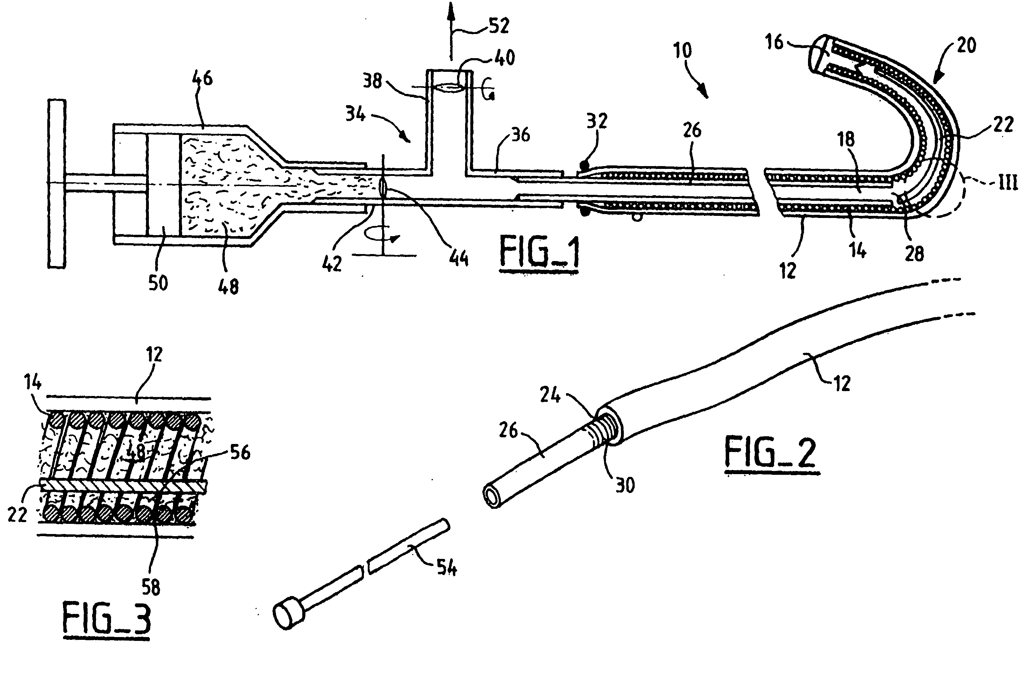

Sur la figure 1, la référence 10 désigne la sonde, qui est du type dont les inconvénients ont été décrits dans les divers articles précités, notamment une sonde auriculaire en forme de J. La forme en J est destinée à faciliter le placement dans l'oreillette droite et elle est obtenue au moyen d'une lame rigide de renforcement inséré à l'intérieur de la gaine isolante (il existe également des techniques de préformage de la gaine isolante et/ou du conducteur interne, mais elles ne présentent pas pour le patient le risque dont il est question dans la présente description).

Plus précisément, sur la figure, la référence 12 désigne la gaine externe, généralement en polyuréthanne, qui enferme un conducteur spiralé 14 se terminant à l'extrémité distale par l'électrode proprement dite 16. Dans le cas d'une sonde bipolaire, celle-ci possède deux conducteurs spiralés, le reste de la structure étant semblable.

La sonde est creuse et son volume intérieur 18 loge, dans la partie distale recourbée 20, une lame métallique de renforcement 22. Il existe d'autres types de sondes qui contiennent le même type de lame, mais située à l'extérieur du canal axial.

La rupture de cette lame peut avoir des conséquences très graves si elle traverse le conducteur spiralé et perce la gaine isolante (dont le matériau, un polyuréthanne, est relativement sensible aux risques de déchirure par un objet métallique dur) et peut, dans certaines configurations, faire saillie contre la paroi de l'oreillette et perforer celle-ci ou l'aorte, avec risque mortel en cas d'hémorragie. Le fragment brisé qui a fait protrusion peut même se détacher de la sonde et être entraîné dans l'artère pulmonaire.

L'extraction d'une telle sonde par traction risque en outre de déchirer la paroi atriale ; par ailleurs, une ablation de la sonde par opération à coeur ouvert reste très risquée. Ainsi, il peut être aussi dangereux, si ce n'est plus dangereux, de tenter l'extraction de la sonde que de la laisser sur place.

Pour résoudre cette difficulté, l'invention propose, essentiellement, d'immobiliser par enrobage la lame métallique par injection d'un matériau durcissable, typiquement un silicone, à l'intérieur de la sonde, ce qui permet d'abandonner sur place la sonde rendue inoffensive ou, si on le souhaite, tenter son explantation avec un risque bien moindre, ou bien encore continuer à utiliser cette sonde.

La première étape consiste à tester l'étanchéité de la sonde. A cet effet, après déconnexion du stimulateur d'avec la partie proximale de la sonde (c'est-à-dire celle portant le connecteur électrique), on branche l'extrémité proximale à un tuyau transparent lui-même relié à une pompe à vide. Après quelques minutes de dépression, si le sang n'est pas remonté, la sonde est considérée étanche et l'on peut poursuivre l'intervention ; dans le cas contraire, on peut envisager une procédure classique d'extraction, ou bien décider de poursuivre néanmoins l'intervention en dépit de l'étanchéité imparfaite (dans ce dernier cas, on peut, si l'on décide de procéder à l'injection du silicone, suivre sa progression à l'intérieur de la sonde en ayant pris soin de faire précéder cette injection d'une faible quantité d'un liquide radio-opaque).

L'étape suivante consiste, après avoir ôté le système d'aspiration, à sectionner le connecteur d'avec la partie proximale de la sonde à une distance connue du connecteur, de manière à pouvoir déterminer avec précision la longueur exacte subsistante de la sonde.

Par l'ouverture 24 ainsi pratiquée, on introduit dans le canal intérieur de la sonde un tube 26 tel qu'un tube métallique flexible, par exemple un tube de 0,5 mm * 0,4 mm * 500 mm en acier inox 304 étiré dur à bouts ébavurés de Microfil SA.

Une variante consiste à ne pas sectionner le connecteur et mettre un raccord étanche sur ce connecteur, puis à relier ce raccord à un moyen d'injection tel que décrit plus bas.

Pour cette introduction, le praticien peut s'aider d'un guide en forme de stylet introduit dans la gaine et dont l'extrémité distale, arrondie, fera légèrement saillie de l'extrémité distale 28 du tube et permettra l'introduction plus aisée de ce dernier. Le tube 26 est introduit sur une longueur telle son extrémité distale 28 vienne approximativement déboucher au voisinage de la lame métallique 22. Cet ajustement de longueur peut avantageusement être obtenu grâce à un repère de couleur 30 (figure 2) que l'on amènera à l'endroit de l'ouverture 24 jusqu'à ce qu'il soit partiellement caché par la gaine 12, indiquant que l'extrémité distale du tube 26 se trouve à proximité de la lame 22.

À ce stade, le praticien réalise l'étanchéité à l'extrémité proximale de la sonde, en ligaturant (en 32) la gaine extérieure 12 sur le tube intérieur 26. Ceci assure également un blocage en translation de ce tube 26 par rapport à la sonde.

On place alors sur l'extrémité proximale saillante du tube 26 un raccord en T 34 (par exemple une vanne trois voies polycarbonate à embout Luer mâle de Bioblock, réf. A13600) dont une première branche 36 est reliée de façon étanche au tube 26, une deuxième branche 38 peut être sélectivement obturée par une vanne manoeuvrable 40 et la troisième branche 42 peut être sélectivement obturée par une vanne manoeuvrable 44. Initialement, les vannes 40 et 44 sont en position d'obturation.

On relie la branche 42 à une seringue 46 (par exemple une seringue 3 cc de EFD, réf. 5109AUV-B) contenant un matériau durcissable tel qu'une résine silicone 48, qui pourra être injectée par action sur un piston 50.

Cette résine est par exemple une colle médicale silicone Raumedic SI 1511 de Rehau, mélangée à un additif de type toluène ou à action équivalente pour augmenter sa fluidité et de préférence chargée de particules radio-opaques permettant de suivre le processus sous amplificateur de brillance et de contrôler *in fine* l'enrobage complet de la lame métallique en confirmant l'étanchéité de la gaine.

La vulcanisation de ce silicone le fait passer d'un état liquide-visqueux à un état solide-souple, ce dernier état correspondant à une dureté Shore d'environ 30 à 80.

Cette injection pourra être réalisée soit par un pistolet actionné à la main par l'opérateur (par exemple un pistolet d'injection *Dispens-Gun* 3 cc de EFD, ref. DG3) , soit par un système automatique à pression asservie, qui présente l'avantage de la possibilité de contrôler d'après le temps d'écoulement la progression de l'injection dans le tube (par exemple un applicateur-doseur temporisé de 0,07 à 7 bars de EFD, ref. 1500 XL avec adaptateur pour seringue 3 cc, ref. 1000Y5152-6).

Tout d'abord, on relie la branche 38 à une source de vide (schématisée par la flèche 52) et on ouvre la vanne 40. Ceci a pour effet de créer un vide à l'intérieur de la sonde, dont on se rappelle que l'on a préalablement vérifié l'étanchéité. Ce vide facilitera l'écoulement du matériau durcissable et évitera la formation de bulles d'air.

On ferme ensuite la vanne 40 et on ouvre la vanne 44, ce qui a pour conséquence, par effet de dépression, d'aspirer le matériau durcissable 48 à travers le tube 26 puis à travers la partie distale, enrobant ainsi la lame métallique 22 sur toute sa longueur. Si la perte de charge est trop importante, on peut parfaire le remplissage en actionnant le piston 50, et même compacter légèrement le matériau en poussant un mandrin 54 dans le tube 26, après avoir retiré le piston 50, en prenant auparavant la précaution de fermer la vanne 40, ou de retirer le connecteur 34.

Avantageusement, avant son introduction, le tube 26 peut être maintenu à faible température (c'est-à-dire à une température sensiblement inférieure à la température ambiante ou à la température du corps, par exemple de l'ordre de +2 à +5°) pour garder un maximum de fluidité au silicone.

En complément, et par mesure de sécurité, il est possible de recouvrir la sonde au préalable d'une deuxième gaine, de manière à assurer une protection supplémentaire par l'extérieur.

L'intervention se termine en attendant la réticulation du silicone. Pour accélérer cette réticulation, on peut éventuellement injecter au préalable un peu d'eau à l'extrémité du tube et/ou en insérant après l'injection un axe préchauffé de dimensions extérieures homologues de celles du tube, la chaleur étant transférée à la résine par les spires métalliques du conducteur.

En fin d'intervention, la lame métallique 22 est enrobée et immobilisée. On notera en outre qu'il y a non seulement remplissage de l'espace situé entre la lame et les spires du conducteur (comme illustré en 56 sur la figure 3), mais également remplissage interstitiel entre les spires (en 58) et même entre les spires et la gaine, ce qui procure un effet d'encastrement mécanique en plus du collage de la lame.

On évite ainsi tout risque d'expulsion de la lame au travers de la gaine et donc de perforation du coeur.

Il suffit alors de ligaturer le bouchon de silicone à l'extrémité proximale de la sonde et d'abandonner celle-ci sur place, à moins que l'on ne souhaite procéder à son explantation, ou encore tenter de la réutiliser, en la raccordant à un adaptateur approprié (par exemple un XT3 de ELA Médical) pour remplacer le connecteur sectionné.

Dans tous les cas de figure, on bénéficie d'une sonde réparée dont la partie distale n'a pas perdu sa flexibilité ; le fluide utilisé, une fois réticulé, garde en effet une souplesse maximale et l'on ne crée pas de nouvelles zones fragiles.

## Revendications

1. Un nécessaire pour la passivation d'une sonde cardiaque installée chez un patient, cette sonde (10) comprenant une extrémité proximale et une extrémité distale (20), un conducteur interne (14), une gaine externe (12), un volume interne (18) s'étendant de l'extrémité proximale à l'extrémité distale, un élément rapporté (22) disposé à l'extrémité distale et dont la rigidité est supérieure à celle, combinée, de la gaine et du conducteur le nécessaire comportant :
― un matériau durcissable (48) susceptible de passer d'un état liquide à un état solide;
― une seringue (46) avec une chambre d'injection pour recevoir le matériau durcissable dans son état fluide, et un orifice de sortie ; et
― un tube d'injection (26) avec une extrémité proximale et une extrémité distale, ce tube d'injection pouvant être inséré dans le volume intérieur de la sonde ;
le nécessaire **caractérisé en ce qu'**il comprend
― un raccord en T (34) avec une première branche (36), une deuxième branche (38) et une troisième branche (42), la deuxième et la troisième branche possédant chacune une valve (40, 44) permettant d'ouvrir et de fermer cette branche;
où la première branche (36) du raccord en T se raccorde à frottement avec l'extrémité proximale du tube d'injection, la deuxième branche (38) du raccord en T est conçue pour être raccordée à une source de vide (52) et la troisième branche (42) du raccord en T se raccorde à l'orifice de sortie de la seringue, et où la première et la seconde valves peuvent être actionnées pour créer un vide dans le volume intérieur de la sonde et aspirer ainsi le matériau durcissable jusqu'à l'extrémité distale de la sonde.

2. Le nécessaire de la revendication 1, comprenant en outre un mandrin (54) dimensionné de manière à lui permettre de passer dans le volume intérieur de la gaine.

3. Le nécessaire de la revendication 1, comprenant en outre une ligature (32) permettant de raccorder la gaine au tube d'injection.

4. Le nécessaire de la revendication 1, où le tube d'injection comporte en outre une marque (30) correspondant à une distance entre cette marque et l'extrémité distale du tube.

5. Le nécessaire de la revendication 1, comprenant en outre une longueur de tube pour relier la deuxième branche du raccord en T à une source de vide.

6. Le nécessaire de la revendication 5, où la longueur de tube est transparente.

7. Le nécessaire de la revendication 1, comprenant en outre un mandrin (54) dimensionné de manière à lui permettre de passer dans le volume intérieur du tube d'injection.

## Patentansprüche

1. Zubehör für die Passivierung einer Herzsonde, die bei einem Patienten installiert ist, wobei die Sonde (10) eine proximale Extremität und eine distale Extremität (20) umfasst, einen inneren Leiter (14), eine externe Hülle (12), ein inneres Volumen (18), welches sich von der proximalen Extremität zur distalen Extremität erstreckt, ein umgeschlagenes Element (22), welches an der distalen Extremität angeordnet ist und dessen Steifigkeit größer als diejenige ist, kombiniert aus der Hülle und dem Leiter, wobei das Zubehör aufweist:
- ein aushärtbares Material (48), welches in der Lage ist, von einem flüssigen Zustand in einen festen Zustand überzugehen;
- eine Spritze (46) mit einer Injektionskammer, um das aushärtbare Material in seinem flüssigen Zustand aufzunehmen, und eine Ausgangsöffnung; und
- eine Injektionsröhre (26) mit einer proximalen Extremität und einer distalen Extremität, wobei die Injektionsröhre in das innere Volumen der Sonde eingeführt werden kann;
wobei das Zubehör **dadurch gekennzeichnet ist, dass** es aufweist:
- eine T-Verbindung (34) mit einem ersten Zweig (36), einem zweiten Zweig (38) und einem dritten Zweig (42), wobei der zweite und der dritte Zweig jeder ein Ventil (40, 44) besitzt, welches den Zweig zu öffnen und zu schließen erlaubt;
wobei der erste Zweig (36) der T-Verbindung in Reibung sich verbindet mit der proximalen Extremität der Injektionsröhre, der zweite Zweig (38) der T-Verbindung vorgesehen ist, um mit einer Vakuumquelle (52) verbunden zu werden, und der dritte Zweig (42) der T-Verbindung mit der Ausgangsöffnung der Spritze verbunden wird, und wobei das erste und das zweite Ventil betätigt werden können, um ein Vakuum in dem inneren Volumen der Sonde zu schaffen und das aushärtbare Material anzusaugen bis zur distalen Extremität der Sonde.

2. Zubehör gemäß Anspruch 1, weiter aufweisend einen Dorn (54), dimensioniert derart, dass er in das innere Volumen der Hülle eintreten kann.

3. Zubehör gemäß Anspruch 1, weiter aufweisend eine Ligatur (32), welche erlaubt, die Hülle mit dem Injektionsrohr zu verbinden.

4. Zubehör gemäß Anspruch 1, wobei die Injektionsröhre des Weiteren eine Marke (30) aufweist, welche einer Distanz zwischen der Marke und dem distalen Ende der Röhre entspricht.

5. Zubehör gemäß Anspruch 1, weiter aufweisend eine Röhrenlänge zum Verbinden des zweiten Zweigs der T-Verbindung mit einer Vakuumquelle.

6. Zubehör gemäß Anspruch 5, wobei die Röhrenlänge transparent ist.

7. Zubehör gemäß Anspruch 1, weiter aufweisend einen Dom (54), derart dimensioniert, dass er in das innere Volumen der Injektionsröhre eintreten kann.

## Claims

1. A kit for passivation of a cardiac lead installed in a patient, said lead (10) having a proximal end and a distal end (20), an interior conductor (14), an exterior sheath (12), an internal volume (18) extending from the proximal end to the distal end, an added element (22) disposed in the distal end and the rigidity of which is greater than the combined rigidity of said sheath and conductor, the kit comprising:
- a solidifiable material (48) that may change from a liquid state to a solid state;
- a syringe (46) having an injection chamber to receive the solidifiable material in its fluid state, and an output port; and
- an injection tube(26) having a proximal opening and a distal opening, said injection tube being insertable in the interior volume of said lead;
said kit being **characterized in that** it includes:
- a T-connector (34) having a first branch (36), a second branch (38)and a third branch (48), the second and third branches each having a valve (40, 44) for opening and closing said branch;
wherein the T-connector first branch (36) frictionally connects to the proximal end of the injection tube, the T-connector second branch (38) is adapted to be connected to a vacuum source (52), and the T-connector third branch (42) connects to the syringe output port, and wherein the first and second valves are operable to create a vacuum in the interior volume of the lead and accordingly aspirate the solidifiable material up to the distal end of the lead.

2. The kit of claim 1, further comprising a stylet (54) having dimensions suitable to pass within the interior volume of the sheath.

3. The kit of claim 1, further comprising a ligature (32) to connect the sheath to the injection tube.

4. The kit of claim 1, wherein the injection tube further comprises a mark (30) corresponding to a distance between said mark and the distal end of the tube.

5. The kit of claim 1, further comprising a length of tubing to connect the second branch of the T-connector to a vacuum source.

6. The kit of claim 5, wherein the length of tubing is transparent.

7. The kit of claim 1, further comprising a stylet (54) having dimensions suitable to pass within the interior volume of the injection tube.
